# EUROPEAN PATENT APPLICATION

(11) **EP 2 486 913 A1**
(43) Date of publication of application: **15.08.2012**
(21) Application number: 11154395.5
(22) Date of filing: 14.02.2011
(51) Int. Cl.: A61K 9/00, A61K 9/70, A61K 31/551

(54) **Rapidly disintegrating oral film formulation for Olanzapin**

(71) Applicant: Labtec GmbH, 40764 Langenfeld (DE)
(72) Inventor: Breitenbach, Armin, 51371 Leverkusen (DE); Schwier, Nina, 52076 Aachen (DE)
(74) Representative: von Renesse, Dorothea

(57) **Abstract**

An orally disintegrating film (ODF) which comprises olanzapine or a salt or solvate thereof, polyvinyl alcohol, polyethylene glycol, one or more plasticizers and whereas at least one of the plasticisers is not PEG1000.

## Description

### FIELD OF THE INVENTION

The invention relates to an oral film formulation comprising olanzapine, its production and use for the treatment of CNS disorders.

### BACKGROUND OF THE INVENTION

Olanzapine is a psychotropic agent that belongs to the thienobenzodiazepine class. The chemical name is 2-methyl-4-(4-methyl-1-piperazinyl)-10H thieno[2,3-b][1,5]benzo-diazepine. The chemical structure of olanzapine is shown in Formula l.

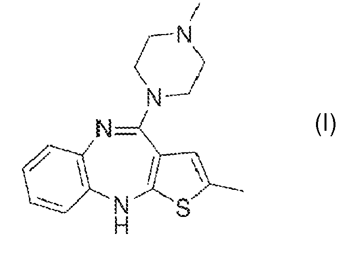

Olanzapine is approved for the treatment of psychotic disorders such as schizophrenia, acute mania in bipolar disorder and agitation associated with schizophrenia and bipolar disorder.

Olanzapine is a selective monoaminergic antagonist with high affinity binding to serotonin, dopamine, histamine and alpha adrenergic receptors. Olanzapine is an antagonist with moderate affinity binding for serotonin 5HT₃ and muscarinic M1-5 receptors. Furthermore olanzapine binds weakly to gamma aminobutyric acid type A (GABA_{A}), benzodiazepine (BZO), and (beta) adrenergic receptors (Ki » 10 µM).

Pharmaceutical dosage forms, such as orally disintegrating film formulations hereinafter ODF, which disintegrate rapidly in the mouth, are advantageous in a wide variety of aspects; in particular they facilitate the oral administration of medicaments to patients who have problems in swallowing or suffer from nausea or emesis. For these patients swallowing conventional oral administration forms such as film-coated tablets is often cumbersome. Due to the rapid disintegration of the film formulation within the buccal cavity the active ingredient from film formulation can be incorporated either over the oral or gastric mucosa. Accordingly they can be designed to enable either gastrointestinal or buccal adsorption of the active ingredient.

Orally disintegrating tablets containing olanzapine are known and sold under the trade name Zyprexa® (VeloTab™). They are prepared by a freeze-drying technique, as is for example described in EP 435 684 A1. A rapidly disintegrating oral film comprising olanzapine is known from WO 2007/009801 A1.

It has now been found by the inventors of the present invention that within oral films the chemical stability of olanzapine is unsatisfying. It particular it was observed that within oral films olanzapine to a certain extend degrades to olanzapine lactam or other unwanted degradation products, which are pharmacologically less effective or ineffective. These degradation products or metabolites impede the quality of the olanzapine containing pharmaceutical composition.

Thus, it is the objective of the present invention to provide an orally disintegrating film and a formulation therefore containing olanzapine, wherein olanzapine exhibits an increased chemical stability.

### SUMMARY OF THE INVENTION

This objective is solved by the orally disintegrating film according to claims 1. Advantageous embodiments of the invention are subject matter of dependent claims. Furthermore the invention relates to a production process of the olanzapine film and to a coating composition suitable for producing said film.

The orally disintegrating film (ODF) according to the invention comprises
(a) olanzapine or a salt or solvate thereof;
(b) polyvinyl alcohol;
(c) polyethylene glycol;
(d) one or more plasticizers;
   whereas at least one of the plasticizers is not PEG1000.

The invention is based on the finding that in the presence of a plasticizer the chemical stability of olanzapine within the film and the film forming composition is increased. With other words: At the presence of a plasticizer the total amount of olanzapine degradation products or metabolites within the composition is markedly reduced. In particular the total amount of the lactam of olanzapine is reduced compared to an identical olanzapine-containing film without a plasticizer.

The lactam compound 2-methyl-5,10-dihydro-4H-thieno[2,3-b][1,5]benzo-diazepin-4-one is a well known degradation product of olanzapine, wherein the 4-methyl piperazine ring is cleaved of from diazepine ring thereby giving rise to the formation of the lactam group (see formula II).

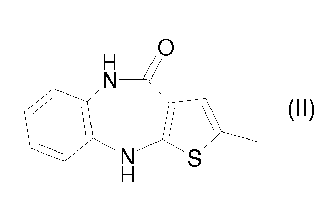

Furthermore the film according to the invention exhibits favourable mechanical properties, in particular a high flexibility while retaining sufficient tensile strength.

The olanzapine ODF according to the invention exhibits one or more of the following advantages over classical dosage forms:
- The olanzapine ODF is more stable, durable and faster dissolving than other conventional dosage forms.
- The ODF is flexible and can be bent or even folded. This prevents inadvertent breakage of the film and distinguishes the ODF from tablets (especially ODTs), which are friable at room temperature and can easily break during transportation or handling by the consumer. Furthermore, unlike some ODTs, the ODF exhibits less moisture sorption, hardening, and slowing down of disintegration over the course of shelf-life due to its properties and packaging configuration.
- The ODF is not friable at room temperature. This attribute prevents fragmenting during transportation under regular conditions and handling and reduces the possibility of residue remaining in the blister or foil pouch as is often observed for some types of ODTs (eg, lyophilized or effervescent ODTs). The lack of friability means the entire intended dose can be accurately consumed by the user, and an individually packaged unit does can be conveniently carried around without risk of product damage.
- The olanzapine ODF enables improved dosing accuracy relative to liquid formulations since every film strip is manufactured to contain a precise amount of the drug.
- The technology for making the ODF is simple and pharmaceutically elegant compared to relatively complex ODT technologies, such as lyophlized, compression molded, effervescent, and sugar floss dosage forms.
- The ODF dosage form allows discrete consumption of the dose, and the smaller size of the dosage form allows for shorter residence time within the oral cavity and potentially more effective avoidance of unpleasant taste.
- The olanzapine ODF not only ensures more accurate administration of drugs but also can improve compliance due to the intuitive nature of the dosage form and its inherent ease of administration. These properties are especially beneficial for the psychotic and/or geriatric disease patients where proper and complete dosing can be difficult.
- ODF's ability to dissolve rapidly without the need for water provides an alternative to patients with swallowing disorders and to patients suffering from nausea, such as those patients receiving chemotherapy.
- Due to the intuitiveness of the concept (the ODF could not be swallowed like a tablet even if one tried) no re-education of the patients is necessary for ODF-administration allowing a rapid uptake of this product in the market.
- Due to the very slim format can be easily carried in small containers, e.g. in the wallet.

Favourably, the film of the invention is non-mucoadhesive.

In sum, the olanzapine ODF combines the benefits of liquid dosage forms (easy administration) and of solid dosage forms (exact secure dosing).

### DEFINITIONS

As used through the present application the following terms have the meaning indicated unless otherwise indicated:
The term "non-mucoadhesive" means that the dosage form does not adhere to a large extend to the mucosal surface of the buccal cavity Furthermore this means, that the film is particularly suitable for the resorption of the active ingredient contained therein in the gastrointestinal tract of the subject.

A "film" according to the invention is defined as a two-dimensional layered or substantially plane structure. The film favourably has a length to thickness ratio ("UT ratio") of at least 40. Within this formula the length is defined as the greatest length extension of said layered or flat structure. Hence, a film with a length of 20 mm, a width of 7.5 mm and a thickness of 0.2 mm has an UT ratio of 100.

A "film formulation" or a "film forming formulation" is any composition which is capable of forming a film. The film can be prepared from the film formulation by various methods, including drying or curing. A "film-forming polymer" refers to a polymer that can form a continuous film upon evaporation of the solvent or carrier and/or upon cure of the polymer.

The term "disintegration" as used in the above definition has its usual and customary meaning in the pharmaceutical arts, as described in <701> of the U.S. Pharmacopoeia (2005 USP/NF) for uncoated tablets, using a basket rack assembly operating at 30 cycles per minute through a distance of 5.5 cm, in a disintegration medium at 37°C. When disintegration requirements are discussed herein, they are preferably met under the foregoing testing conditions, at a pH of 4.0 or 6. A film or other dosage form is said to be "disintegrated" if it is completely disintegrated, a state in which any residue of the unit remaining on the screen of the test apparatus, or in the mouth, is a soft mass having no palpably film core, or fragments of a tablet coating or capsule shell. Disintegration thus does not imply complete dissolution of the dosage unit or even the active constituent, although a dissolved dosage unit would typically be completely disintegrated. When reference to Ph. Eur. 2.9.1 (disintegration) is made herein, it will be understood that the disintegration conditions described above under <701> USP can also be employed.

The term "plasticizer" as used herein is meant to designate a component or a mixture that increases the plasticity. The increased plasticity can be measured by an increased strain elongation of the film. The strain elongation can be measured in a static tension test. In this test the film is subjected to increased tension until the film breaks. The elongation at the breaking point is designated as strain elongation (change in length per unit length).

The term "stability" as applied to the active ingredient is the capacity of the active ingredient (here: olanzapine) to remain within the pharmaceutical composition within the established specifications, i.e. to substantially maintain its identity, strength, quality and purity throughout the retest or expiration dating period. As applied for the instant invention the stability is assessed in an accelerated stability study of the final pharmaceutical composition (the drug product), namely the film is subjected to stress conditions of 60°C over a period of 15 days.

In the context of the invention the term "degradation product", which is used interchangeable with "metabolites" of the olanzapine relates to any molecular compound resulting from changes of the olanzapine brought about over time and/or by the action of, e.g., light, temperature, pH, water, or by reaction with any other compound in the composition. Such changes may occur as a result of manufacture and/or storage or metabolisation (e.g., deamidation, oxidation, aggregation) of the olanzapine containing composition.

The term "treatment" or "treating" refers to administration to an individual of an agent of interest wherein the agent alleviates, reduces, mitigates or prevents any pathology or disorder of a subject (in particular a human) suffering there from. The term "treatment" therefore includes prophylaxis and prevention of disorders.

The term "pharmaceutically acceptable salts" is used to describe those salts in which the anion (or cation) does not contribute significantly to the toxicity or pharmacological activity of the salt, and, as such, they are the pharmacological equivalents of the bases of the compounds to which they refer. Examples of pharmaceutically acceptable acids that are useful for the purposes of salt formation include but are not limited to hydrochloric, hydrobromic, hydroiodic, citric, acetic, benzoic, mandelic, fumaric, succinic, phosphoric, nitric, maleic, mucic, isethionic, palmitic, tannic and others. The active salt combinations of the pharmacologic ingredients may be the free acids, bases or as salts having anionic functional groups such as bitartrate, maleate, citrate, chloride, bromide, acetate and sulfate. The source of the functional groups may be natural or synthetic.

"Therapeutically effective dose", "therapeutically effective amount" or "an effective amount" refers to an amount of an active ingredient that is useful for treating migraine, cluster headache, headache or altitude sickness.

Unless specified otherwise, the term "wt%" as used herein relates to the relative amount by weight with respect to the final (dried) pharmaceutical product (as opposed to the composition which is used for providing it). It this denotes the percentage of the total dry weight contributed by the respective component.

When doses are given for a drug and its salt, it will be understood that the calculated dose is based on the molecular weight of the active pharmaceutical ingredient, which includes the cationic and anionic species in the case of a salt, and just the base when the active principle is not present as a salt. In addition, when reference is made to the salt of a drug and pharmaceutically acceptable salts thereof, it will be understood that salts of the base form of the base drug are intended.

When ranges are given by specifying the lower end of a range separately from the upper end of the range, it will be understood that the range can be defined by selectively combining anyone of the lower end variables with anyone of the upper end variables that is mathematically possible.

### DETAILLED DESCRIPTION OF THE INVENTION

As outlined above the invention relates to an olanzapine containing pharmaceutical composition with an increased stability of olanzapine, i.e. a reduced content of olanzapine degradation products. The stability of olanzapine is increased by the presence of a plasticizer.

It has been found that even after the conditions of a stress test, the content of the olanzapine degradation products is low compared to the control. The film according to the invention comprises no more than about 0.5 wt%, preferably no more than 0.3 wt%, most preferred no more than 0.2 wt % of olanzapine degradation products after the film has been subjected to an accelerated stability test (6 days storage at 60°C).

The enhanced stability of the film particularly applies for the olanzapine lactam as the most critical degradation product. Thus, the film according the invention comprises no more than about 0.3 wt%, preferably no more than 0.2 wt%, most preferred no more than 0.1 wt% of olanzapine lactam after the film has been subjected to an accelerated stability test (6 days storage at 60°C).

The olanzapine can be present in form of a salt or solvate thereof. Examples of useful salts are the addition salts with an inorganic acid, selected from hydrochloric, hydrobromic, sulphuric, nitric, and phosphoric acid; or with an organic acid selected from acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, p-aminosalicylic, and pamoic acid. Examples of suitable salts are also described in EP 0 454 436 B1.

The olanzapine can also be present in an anhydrous form, such as those disclosed in EP 0 733 653 B1, which are designated therein as Form I and Form II; in US 6,348,458 designated therein as Form III, Form IV, Form V; in US 2002/0086993 A1, designated therein as Form X.

Olanzapine can be present in form of a specific polymorph. It can be applied as the polymorphic form A or in the form of olanzapine solvates, such as the acetonitrile/methylene chloride/water, and acetonitrile/water mixed solvates, and the 2-propanol solvate, methylene chloride solvate IA, methylene chloride solvate 1 B as disclosed in WO 01/47933. The most preferred polymorphic form is the polymorph I.

Also useful are the solvates disclosed in EP 0 733 634, and are designated therein as mono methanol solvate, mono ethanol solvate, and mono 1-propanol solvate. Also useful are hydrates of olanzapine which are disclosed e.g. in EP 0 831 098 B1, and are designated therein as monohydrate I and dihydrate I.

It is preferred that the film according to the invention comprises 1 to 40%, preferably 5 to 30%, more preferably 10 to 25% and most preferred 18 to 22% by weight of olanzapine or a salt or solvate thereof.

In a further aspect of the invention the film of the invention the olanzapine is given as a dispersion within said film.

In one embodiment of the invention the film comprises a polyvinyl alcohol of 20-400 KDa and having a degree of hydrolysis of 85 to 90%, and most preferably a polyvinyl alcohol (PVA) of about 31 KDa having a degree of hydrolysis of 68.7 to 88.8 %. Such PVA is known as the market product Mowiol 4-88.

In a further embodiment of the invention the polyethylene glycol polymer (PEG) of the film is a combination of two polyethylene glycol polymers, preferably a combination of at least one solid PEG and at least one liquid PEG (both at room temperature), more preferably a combination of a PEG with a molecular weight between 3000 and 6000 Da and a PEG with a molecular weight between 250 and 1000 Da, and specifically a combination of PEG400 and PEG4000. PEGs which are used for the preparation of drugs are known as the commercial product Macrogol (with Macrogol 400 being PEG400, Macrogol 4000 being , PEG4000).

The film according to the invention comprises a plasticizer whereas said plasticizer. In a preferred embodiment the plasticizer is a organic compound having at least one hydroxyl group, which is preferably an alcohol. According to the invention said alcohol can be a mono-, di, tri or polyalcohol.

In a further aspect of the invention said plasticizer is an organic compound having at least an ether group.

In a preferred embodiment of the invention said plasticizer is an alcohol or an ether, either monomeric or polymeric which has an ratio of C-atoms to O-atoms between 1:1 and 2:1.

In a further embodiment said plasticizer is a polyether with hydroxyl groups.

In a more preferred embodiment the plasticizer is selected from the group consisting of mono-, di- or polysaccharides, polyether, polyvalent alcohols and esters thereof. In a most preferred embodiment of the invention said plasticizer is selected from the group consisting of sorbitol, glycerol, xanthan, a medium chain triglyceride, polyethylene glycol or carrageenan. The most preferred plasticizer is glycerol.

It one embodiment of the invention the film comprises 1 to 20%, preferably 1 to 10%, more preferably 2 to 8% and most preferred 3 to 6% by weight of plasticizer.

In one aspect of the invention an addition of 10 wt% of plasticizer to the film increases the strain elongation by more than 1%, preferably more than 2% and more preferably more than 3% when compared to the film without plasticizer.

Moreover, the film usually comprises a disintegrant, which is preferably starch or a starch derivative, more preferably rice starch.

In a further aspect of the invention the film has a thickness between 10 and 500 µm, preferably between 20 and 200 µm and most preferably between 50 and 100 µm. In order to avoid an unpleasant sensation in the mouth, the film thickness must not be too great.

In one aspect of the invention, the film has a weight of from about 20 to about 100 milligrams, preferably from about 25 to about 80 milligrams. The film may vary in surface area and preferably does not exceed 10 or 12 cm² in surface area.

In a further aspect of the invention the film has a ratio of UT ratio being at least 40, preferably at least 100, more preferably at least 200, and even more preferably at least 500.

In another aspect of the invention the film has a tensile strength between 2 and 20 N/15mmm, preferably between 2 and 10 N/15mm and more preferably between 2 and 5 N/15mm. The tensile strength of the film is suitably determined according to the DIN standard DIN EN ISO 527-1 and 527-3. Alternatively the determination of the tensile strength can be performed according the ASTM international test method for thin plastic sheeting D 882-02.

The residual water content of the film is between 2% and 10% of water by weight, preferably between 2% and 6% water and more preferably between 2% and 4%.

In a further aspect of the invention the oral film comprises one or more additives. Additives include, but are not limited to effervescent agents, tensides, sweetening agents, flavouring agents, taste masking agents, colouring agents, colourants and fillers.

In one particular embodiment of the invention the additive fraction of 1-15 wt% within the oral film comprises:
(a) 0 to 10 wt% sweetening agent
(b) 0 to 10 wt% flavouring agent
(c) 0 to 10 wt% colouring agent

In one particular embodiment the additive fraction comprises:
(a) 0.25 to 4 wt% sweetening agent
(b) 1 to 8 wt% flavouring agent
(c) 1 to 8 wt% colouring agent

In one aspect of the invention the formulation provides a film which consists of a single layer, whereby the layer preferably has a homogenous structure. Preferably, the film is a single-layered homogeneous film. The film can have a rigid or a flexible consistency, the latter one is preferred.

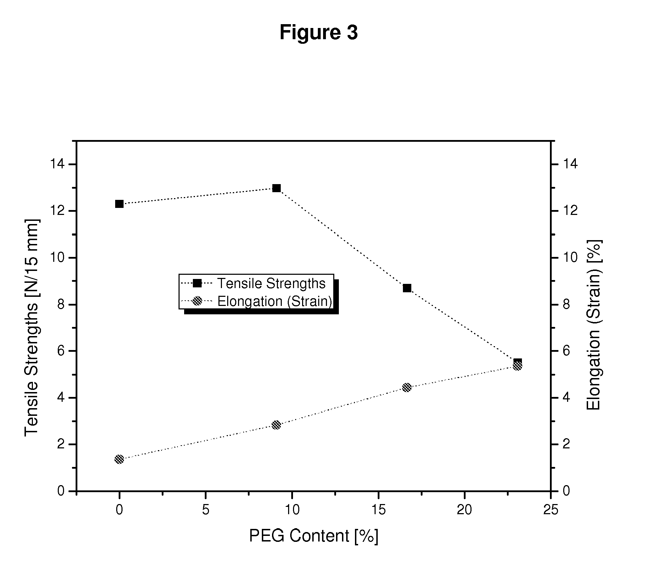

In a further aspect of the invention the rheological properties, particularly the tensile strength and the elongation of the ODF can be controlled by the PEG content. As the inventors found out, the elastic behaviour and the flexibility increased linearly with increasing amount of PEG. In parallel the tensile strength decreased when the PEG content of the film exceeded a certain threshold content (see Figure 3).

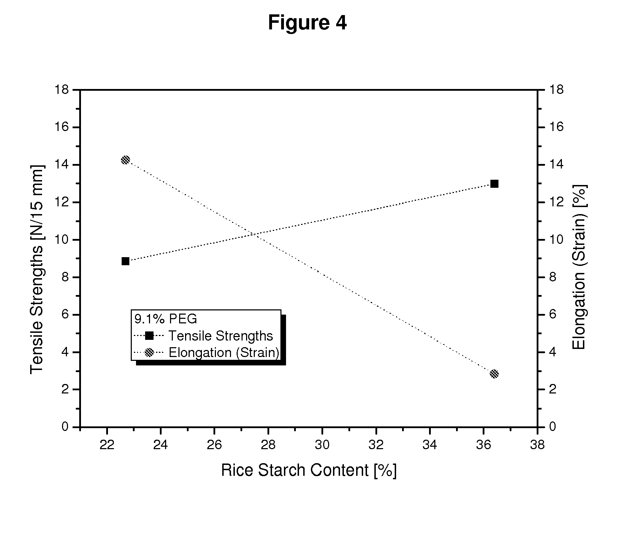

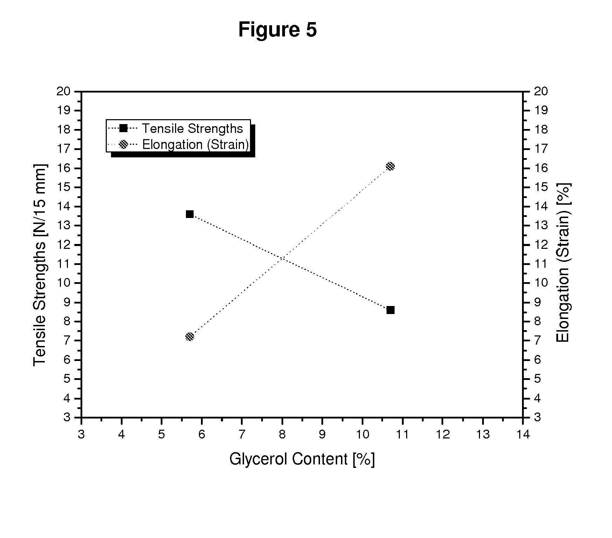

In another aspect of the invention the rheological properties of the ODF can bei controlled by the addition of a filler, such as rice starch (s. Fig. 4) and/or a plasticizer, such as glycerol (s. Fig. 5). Said two classes of compounds show inverse effects with regard to rheological properties. With increasing amount of filler the flexibility is found to be decreased and the strength is found to be increased.

By appropriate adjustment of PEG, filler and plasticizer the rheological properties can be tailored to the specific needs.

In the most preferred embodiment of the invention the ODF formulation comprises of 44 % (w/w) PVA as base matrix polymer, 12 % (w/w) of PEG, 4 % (w/w) of glycerol and 20 % (w/w) of rice starch, since this composition showed a good balance of rheological properties and fast dissolution behavior.

In one aspect of the invention the formulation provides a non-mucoadhesive ODF which is able to disintegrate upon contact with saliva in the buccal cavity. The film preferably disintegrates within about 10, 20, 30 or 60 seconds of administration, after it is swallowed. The prompt disintegration and swallowing of the film helps to assure gastrointestinal absorption of the dosage form.

Preferably, the single-layered film preparation can be substantially free of cavities, a "cavity" being understood as being a region which is filled with a fluid (a gas and/or a liquid). Such a cavity usually has a diameter of less than 100 µm. Preferably, a film-form preparation is substantially free of gas bubbles and/or cavities that contain a fluid (gas and/or liquid).

In a further aspect of the invention the film formulation can be essentially free of tensides. A film that is essentially free of tensides has a tenside concentration of less than 1 wt% related to the complete formulation, preferably less than 0.1 wt%, and more preferably less than 0.01 wt%. In a preferred aspect of the invention the film is produced without addition of tensides as component. The term "tenside" as used herein refers to any substance or compound that reduces surface tension when dissolved in water or an aqueous solution, or that reduces interfacial tension between two liquids, or between a liquid and a solid. Unless otherwise indicated, the term "tenside" includes substances or compounds that are anionic, cationic, nonionic, zwitterionic and/or amphoteric in nature.

In another aspect of the invention the film formulation can be essentially free of effervescent additives. A film that is essentially free of effervescent additives has a concentration of effervescent agents of less than 1 wt% related to the complete formulation, preferably less than 0.1 wt%, and more preferably less than 0.01 wt%. In a preferred aspect of the invention the film is produced without addition of effervescent additives as component. As used herein, an effervescent additive means a material or compound that is capable of rapidly releasing gas upon contact with aqueous solvents, or change in pH value or increased temperature. Effervescent additives are preferably substances that in the buccal cavity, e.g. by contact with the saliva, releases a gas such as CO₂.

In one aspect of the invention the film formulation can be essentially free of taste masking agents. A film that is essentially free of taste masking agents has a concentration of taste masking agents of less than 1 wt% related to the complete formulation, preferably less than 0.1 wt%, and more preferably less than 0.01 wt%. In one aspect of the invention the film is produced without addition of taste masking agents as component. As used herein, a taste masking agent refers to a compound that interacts with a bad or unpleasant (e.g. bitter or sour) tasting substance thereby masking the bad or unpleasant taste.

A taste masking agent is preferably used within formulations to mask the bad taste of an active ingredient. The film formulation is preferably free of ion exchange resins, inclusion compounds formed with cyclodextrin or a cyclodextrin derivative, or coatings of the active ingredient with a layer, e.g. comprising Eudragit. In a preferred aspect of the invention the active ingredient is contained within the formulation in free form and not e.g. encapsulated or enclosed.

Suitable sweeteners that can be included are those well known in the art, including both natural and artificial sweeteners. Suitable sweeteners include, e.g.:
(a) Water-soluble sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin;
(b) Water-soluble artificial sweeteners such as the soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like;
(c) Dipeptide based sweeteners, such as L-aspartic acid derived sweeteners, such as L-aspartyl-L-phenylalanine methyl ester (aspartame) and materials described in U.S. Pat. No. 3,492,131, L-alpha-aspartyl-N-(2,2,4,4-tetramethyl-3-thietanyl)-D-alanin amide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclohexyen)-alanine, and the like;
(d) Water-soluble sweeteners derived from naturally occurring water-soluble sweeteners, such as a chlorinated derivative of ordinary sugar (sucrose), known, for example, under the product description of sucralose; and
(e) Protein based sweeteners such as thaumatoccous danielli (Thaumatin I and II).

In general, an effective amount of auxiliary sweetener is utilized to provide the level of sweetness desired for a particular composition, and this amount will vary with the sweetener selected. This amount will normally be 0.01 wt% to about 10 wt% by weight of the composition when using an easily extractable sweetener. The water-soluble sweeteners described in category (a) above, are usually used in amounts of about 0.01 to about 10 wt%, and preferably in amounts of about 0.5 to about 5 wt%. Some of the sweeteners in category (a) (e.g., glycyrrhizin) can be used in amounts set forth for categories (b) to (e) below due to the sweeteners' known sweetening ability. In contrast, the sweeteners described in categories (b) to (e) are generally used in amounts of about 0.01 to about 10 wt%, with about 0.5 to about 8 wt% being preferred and about 1 to about 5 wt% being most preferred. These amounts may be used to achieve a desired level of sweetness independent from the flavour level achieved from any optional flavour oils used.

The flavourings that can be used include those known to the skilled artisan, such as natural and artificial flavours. These flavourings may be chosen from synthetic flavour oils and flavouring aromatics, and/or oils, oleo resins and extracts derived from plants, leaves, flowers, fruits and so forth, and combinations thereof. Representative flavour oils include: spearmint oil, cinnamon oil, peppermint oil, clove oil, bay oil, thyme oil, cedar leaf oil, oil of nutmeg, orange oil, oil of sage, and oil of bitter almonds. Also useful are artificial, natural or synthetic fruit flavours such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavourings can be used individually or in admixture. Commonly used flavours include mints such as peppermint, artificial vanilla, cinnamon derivatives, and various fruit flavours, whether employed individually or in admixture. Flavourings such as aldehydes and esters including cinnamyl acetate, cinnamaldehyde, citral, diethylacetal, dihydrocarvyl acetate, eugenyl formate, p-methylanisole, and so forth may also be used. Generally, any flavouring or food additive, such as those described in the EU database of flavouring substances, may be used. Further examples of aldehyde flavourings include, but are not limited to acetaldehyde (apple); benzaldehyde (cherry, almond); cinnamic aldehyde (cinnamon); citral, i.e., alpha citral (lemon, lime); neral, i.e. beta citral (lemon, lime); decanal (orange, lemon); ethyl vanillin (vanilla, cream); heliotropine, i.e., piperonal (vanilla, cream); vanillin (vanilla, cream); alpha-amyl cinnamaldehyde (spicy fruity flavours); butyraldehyde (butter, cheese); valeraldehyde (butter, cheese); citronellal (modifies, many types); decanal (citrus fruits); aldehyde C-8 (citrus fruits); aldehyde C-9 (citrusfruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyl-5-heptenal, i.e. melonal (melon); 2-6-dimethyloctanal (greenfruit); and 2-dodecenal (citrus, mandarin); cherry; grape; mixtures thereof; and the like.

The amount of flavouring employed is normally a matter of preference subject to such factors as flavour type, individual flavour, and strength desired. Thus, the amount may be varied in order to obtain the result desired in the final product. Such variations are within the capabilities of those skilled in the art without the need for undue experimentation. In general, amounts of about 0.1 to about 15 wt% are useable with amounts of about 1 to about 10 wt% being preferred and amounts from about 2 to about 5 wt% are more preferred.

The compositions of this invention can also contain colouring agents or colourants. The colouring agents are used in amounts effective to produce the desired colour. The colouring agents useful in the present invention include pigments such as titanium dioxide, which may be incorporated in amounts of up to about 5 wt%, and preferably less than about 1 wt%. Colourants can also include natural food colours and dyes suitable for food, drug and cosmetic applications. These colorants are known as FD&C dyes and lakes. The materials acceptable for the foregoing spectrum of use are preferably water-soluble, and include Indigotine (E132), which is the disodium salt of 2,2'-Bis(2,3-dihydro-3-oxoindolyliden). Similarly, the dye known as Green No. 3 (E143) comprises a triphenylmethane dye and is the monosodium salt of 4-[4-N-ethyl-p-sulfobenzylamino) diphenyl-methylene]-[1-N-ethyl-N-p-sulfonium benzyl)-2,5-cyclo-hexadienimine]. A full recitation of all food dyes and their corresponding chemical structures maybe found in the Kirk-Othmer Encyclopedia of Chemical Technology, Volume 5, Pages 857-884, which text is accordingly incorporated herein by reference.

As fillers there can be used salts, such as carbonates, phosphates, oxides, such as e.g. SiO₂ especially in the form of Aerosil, or the like and/or cellulose and derivatives thereof, and also sparingly soluble sugars and sugar derivatives, such as, for example, lactose or starch derivatives such as cyclodextrins, provided they are in substantially undissolved form in the product and therefore fulfil the mechanical properties of a filler. Preferably, SiO₂ is used as filler. However, in a preferred embodiment of the invention the composition can be devoid of any filler.

In a further aspect of the invention the film is arranged on a carrier foil. Furthermore, the film according to the invention or the preparation according to the invention can be provided with a carrier foil made of polyethylene paper (PE paper), polypropylene foil (PP foil) or polyethylene terephthalate foil (PET foil). Preferably, the film according to the invention or the preparation according to the invention is provided on a carrier foil made of polyethylene paper (PE paper), polypropylene foil (PP foil) or polyethylene terephthalate foil (PET foil).

In a further embodiment of the invention the ODF is packed in a pouch which is opened and removed before application. In a more preferred embodiment the pouch is made of composite foil.

Preferred is a film comprising the following components in the given proportions:
(a) 2 to 35 wt% and preferably 5 to 20 wt% olanzapine or a salt or solvate thereof;
(b) 20 to 75 wt% and preferably 30 to 55 wt% polyvinyl alcohol;
(c) 2 to 15 wt% and preferably 5 to 10 wt% polyethylene glycol, preferably a mixture of PEG 400 and PEG 4000;
(d) 1 to 10 wt% and preferably 2 to 5 wt% plasticizer; preferably glycerol;
(e) 10 to 30 wt% and preferably 15 to 25 wt% disintegrant, preferably rice starch; and
(f) 1 to 15 wt% of additives, such as sweetening agents, flavouring agents, taste masking agents or colouring agents.

Also preferred is a film comprising the following components in the given proportions:
(a) 2 to 35 wt% and preferably 5 to 20 wt% olanzapine or a salt or solvate thereof;
(b) 20 to 75 wt% and preferably 30 to 55 wt% polyvinyl alcohol;
(c) 2 to 15 wt% and preferably 5 to 10 wt% polyethylene glycol, which is not PEG 400, but is preferably PEG 4000;
(d) 1 to 10 wt% and preferably 2 to 5 wt% plasticizer; preferably glycerol;
(e) 10 to 30 wt% and preferably 15 to 25 wt% disintegrant, preferably rice starch; and
(f) 1 to 15 wt% of additives, such as sweetening agents, flavouring agents, taste masking agents or colouring agents.

Preferably, the polyvinyl alcohol listed under (b) is a polyvinyl alcohol of 20-400 KDa and having a degree of hydrolysis of 85 to 90%, and most preferably Mowiol 4-88.

Preferably, the polyethylene glycol listed under (c) is a polyethylene glycol polymer (PEG) of the film is a combination of two polyethylene glycol polymers, preferably a combination of at least one solid PEG and at least one liquid PEG (both at room temperature), more preferably a combination of a PEG with a molecular weight between 3000 and 6000 Da and a PEG with a molecular weight between 250 and 1000 Da, and specifically a combination of PEG400 and PEG4000. In a specific aspect of the invention the liquid PEG is not PEG400. In another aspect of the invention the ODF contains PEG4000 as the only poylethylene glycol polymer.

In a preferred embodiment of the invention the ODF contains PEG4000 as the only poylethylene glycol polymer and glycerol as plasticizer.

When using a combination of a solid and a liquid PEG polymer, it is preferred that the amount of the solid PEG exceeds the liquid PEG. In a more preferred embodiment the film comprises 6 to 15 wt% of the solid PEG and 1 to 8 wt% of the liquid PEG. Preferably, the plasticizer listed under (d) is selected from the group consisting of sorbitol, glycerol, xanthan, a medium chain triglyceride or carrageenan a poly alcohol, more preferably glycerol.

The composition according to the invention is preferably prepared in a process comprising the following steps:
(i) preparation of a homogenous solution/dispersion of the different components in the presence of a solvent;
(ii) applying the solution/dispersion to a carrier
(iii) drying of the applied solution/dispersion
(iv) division into films with a defined area.

It has also been surprisingly found out that the use of water-alcohol mixture as solvent for the coating composition in step (i) results in a film with increased stability of olanzapine. Therefore a process is preferred, which makes use of a water-alcohol mixture as a solvent. Possible alcohols are e.g.: methanol, isopropyl alcohol, propanol, butanol or in general, C₁ - C₈ alcohols. In general, preferred alcohols are isopropylalcohol or ethanol, in particular ethanol.

In the most preferred embodiment the use of a water-alcohol mixture is combined with glycerol as plasticizer for the film of the invention.

In step (i) the different components are mixed in a suitable solvent to obtain a homogenous mixture. This solution can be a monophasic solution, a dispension or an emulsion.

In step (ii) the final coating mixture is applied to a carrier with the aid of a suitable coating method. As carrier material there can be used, for example, PE paper or PP or PET foil. The coated carrier material is dried between 30 to 120°C, preferably between 30 to 70°C.

In step (iii) the coating mixture which has been applied in step (ii) to the carrier is dried to obtain a film with reduced water and/or solvent content. This process is known to the skilled person as "solvent-casting". In a preferred drying process the film is kept for approximately 20 min at a temperature of 30°C to 120°C; preferred at between 50°C to 100°C.

The coated carrier material is then processed further in step (iv) to form separate films of defined area. This can be effected by punching, cutting or stamping. The films are individually packed into sachets with or without carrier foil. They can also be packed into multiple-dose containers. Prior to administration, where applicable the active-ingredient-containing film is removed from the carrier material.

The film of the invention is preferably prepared with a composition comprising the following components in the given proportions:
(a) 2 to 10 wt% olanzapine or a salt or solvate thereof;
(b) 8 to 18 wt% polyvinyl alcohol (e.g. Mowiol 4-88);
(c) 5 to 15 wt% polyethylene glycol, preferably a mixture of PEG 400 and PEG 4000;
(d) 0.5 to 10 wt% plasticizer, preferably glycerol;
(e) 5 to 15 wt% disintegrant, preferably rice starch;
(f) 0.25 to 5 wt% additive, preferably sweetener and flavouring agent;
(g) 40 to 60 wt% water; and
(h) 10 to 20 wt% alcohol, preferably ethanol.

The film formulation is used according to the invention for the administration of olanzapine for treatment of CNS disorders. Preferably, the film formulation comprising olanzapine is used to produce a medicament for the treatment of psychotic disorders, such as schizophrenia, schizophreniform disorder, mild anxiety and acute mania.

### I. EXAMPLE

### 1. Preparation of the olanzapine film

Olanzapine films were prepared for olanzapine doses of 5 mg, 10 mg and 15 mg, respectively. The olanzapine film 5 mg has a size of 3 cm², the olanzapine film 10 mg has a size of 6 cm² and the olanzapine film 15 mg has a size of 9 cm². All three dosages were prepared with the same formulation composition and differ only in their size.

The composition of the dried 3 cm², 6 cm² and 9 cm² films containing glycerol as a plasticiser is summarised in **Table 1**. The composition of the standard batch OLA052 which was used as control is prepared according Table 1 wherein glycerol is omitted and Macrogol 400 and 4000 substituted with Macrogol 1000. The right column depicts the weighing of all raw materials including water and ethanol for a 100kg batch, which has a solids content of about 33%. The dried films contain 1 to 4% residual water (relating to the weight of the dried film).

### 2. Stability analysis

### 2.1 Experimental procedures

For the stability assessment the films were subjected to an accelerated stress conditions as follows. The olanzapine films were incubated in a cabinet dryer for 6 days at 60°C whereby the films were in the standard packaging material. Afterwards the films were dissolved in a mixture of acetonitrile and water (50/50%) and analysed by HPLC for quantification of degradation products.

Analytical method of assay and related substances: The films are dissolved in acetonitrile/water (50/50). The solutions are analyzed by means of RP-HPLC and UV detection at 254 nm.

The impurities are characterized by their respective retention times normalized to the retention time of olanzapine which is set to 1.0.

### Content of known and unknown impurities of olanzapine

Considering the response factors, unknown impurities are set to a response factor of 1.00. All impurities are calculated by a one point calibration using 0.5 % standard olanzapine at 254 nm. All impurities of the active ingredient are applied to olanzapine in [%]. Unknown signals from placebo, solvent or artefacts can be ignored.

### 2.2 Product related degradation products of olanzapine

The degradation of olanzapine resulted in formation of the olanzapine-derived lactam (see formula II) and further unknown degradation products. The quantification of the lactam and the total content of degradation products were used to study several stability-improving strategies.

### 2.3. Analysis of different stability strategies

As a control a standard olanzapine batch (batch number: OLA052) without a plasticizer was stored for 6 month under ICH conditions (40°C and 75% relative humidity) proved to be unstable: The content of the lactam degradation product exceeded the specification limit of 0.50%.

### 2.3.1 Results

It was tested if a reduced water content inhibits the formation of olanzapine degradation products. This was accomplished by increasing the drying temperature and drying time The force-dried base formulation OLA 100 with a residual water content of 2% showed significantly less lactam degradation in the stress test compared to the conventionally dried base formulation OLA 100 with a residual water content of 6% (designated as batch OLA108, see table 2A). However this strategy for improvement requires a more complex production process and harbours a risk for harming the overall product quality.

With regard to a potential stability enhancing effect the following plasticizers were added to the basic recipe or were used in a different amount within the film:
- Glycerol
- Sorbitol
- Xanthan
- Medium-chain triglylceride ester (e.g. Miglyol)
- Carrageenan
- Increase of Macrogol proportion (e.g. Macrogol 400, 1000, 4000)

After stressing the samples for 6 days at 60°C the lactam as the critical degradation product was found to be reduced in all formulations containing one the above listed plasticizers. As an example the reduced degradation in glycerol-containing base formulation (OLA100) as compared to basic formula without any plasticizer (OLA073) is shown in Table 2B. It could be shown that within the film formulation these plasticizers not only enhance the chemical stability but also increase the flexibility/plasticity of the film and thereby increase their mechanical stability. Hence the plasticizers are beneficial for the chemical and the mechanical stability of the film.

Due to the favourable elasticity properties, the glycerol-containing formulation was used for further tests.

As described above, the degradation product lactam is formed to a much lesser extent in the glycerol-containing formulation OLA100 compared to the base formulation OLA073.

Due to process reasons the coating composition for the subsequent development steps, was not prepared with pure water, but with a water-ethanol mixture (75 vol.% + 25 vol.%).

The stability tests revealed that the charges, which were obtained from the water-ethanol mixture, exhibited a further improved stability of olanzapine in the film. In Table 2C, the stability of the batch OLA100, which was prepared from pure aqueous coating composition, and the batch OLA115 obtained with the same recipe from a water-ethanol mixture were compared against each other.

Table 2C clearly shows that the production of aqueous-ethanolic coating material further reduced the degradation of olanzapine in the film. The composition of the batch OLA115 was therefore determined as the final formulation of olanzapine film.

Based on the glycerol-containing film formulation it was tested if an additional reduction of the residual water content will lead to a further enhancement of the stability. The subsequent stress test and analysis of the films showed that the residual water content in the film does not significantly affect the formation of the lactam degradation product. Table 2D shows the comparative results of the batches OLA100 and OLA108. The two batches were made after above described optimized recipe (including glycerol and prepared from a water-ethanol mixture) but differing in their residual water content.

The two formulations exhibit almost identical lactam content. The total amount of degradation products in the batch with 6% residual water content (OLA108) was even lower than in the batch OLA100 which contained only 2% of water. These results show that in the presence of a platicizer there is no need to reduce the residual water content. The degradation of olanzapine is thus reduced primarily by the addition of a plasticizer such as glycerol as well as by the use of a water-ethanol mixture in the production.

### II. EXAMPLE

### 1. Regulation of the rheological properties of the olanzapine film

In order to assess the effect of different ingredients on the rheological properties of the olanzapine ODF, film batches with a PEG content between 0% and 23% were prepared and tested for tensile strength and elongation.

Fig.3 outlines the effect of increasing amounts of PEG on the rheological properties of placebo RapidFilms based on PVA as matrix polymer.

It can be clearly seen that the elastic behavior and the flexibility of the films increased linearly with increasing amount of PEG; yet, amounts significantly exceeding 10% (w/w) caused an increasing loss of mechanical stability (tensile strengths) in parallel.

To achieve an acceptable balance of required flexibility, tensile strength and strain with fast film dissolution and a comfortable tong feeling it was found that the addition of filler was beneficial.

Figure 4 and Figure 5 show the inverse effects of the addition of the filler rice starch (decreasing flexibility and increasing strengths with increasing amount) and the plasticizer glycerol (increasing flexibility and decreasing strengths with increasing amount).

By careful adjustment of the amounts of all these excipients the properties of the resulting films could be tailored to the needs of the ODF application.

### FIGURE LEGENDS

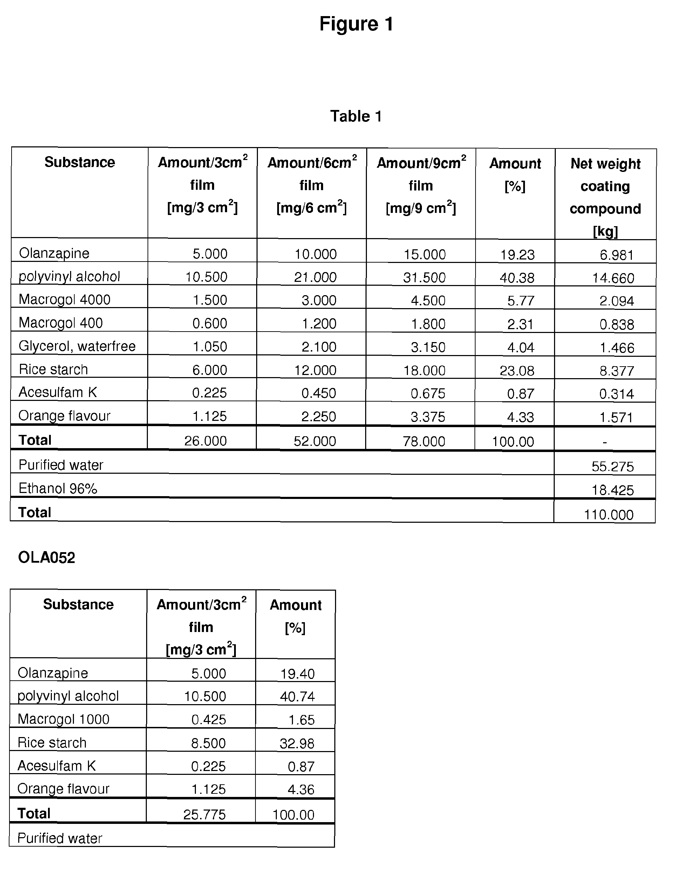

- **Figure 1:**: Figure depicting Table 1 showing composition of olanzapine film containing glycerol as plasticizer (containing 5mg, 10mg or 15 mg olanzapine). The table shows the composition of dried, i.e. water free and solvent free 3 cm², 6 cm² and 9 cm² film patches. In the most right column the net weight of all ingredients including water and ethanol are given for a 110 kg batch, which has a solids content of 33%.

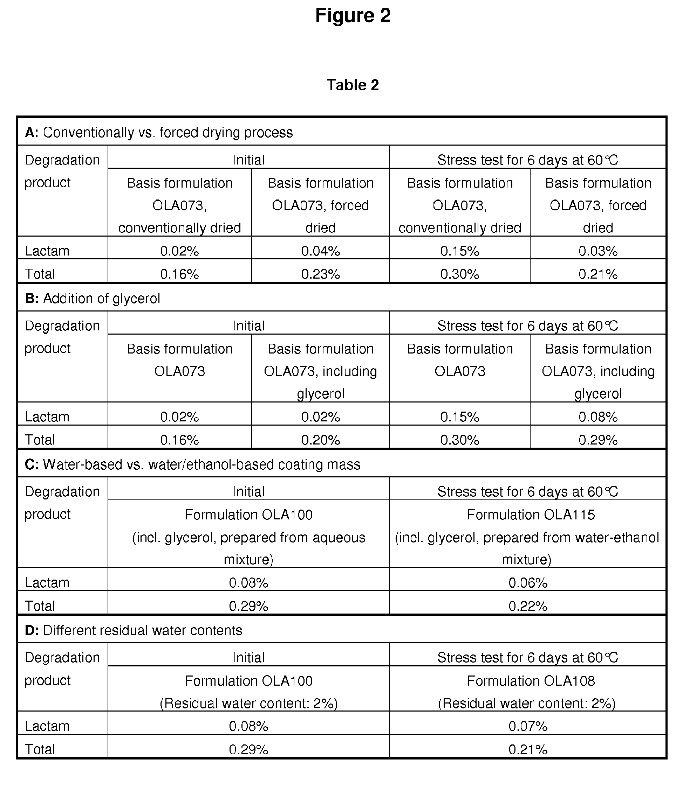

- **Figure 2:**: Figure depicting Table 2 showing stability of different olanzapine films after performing a stress test (6 days for 60°C) **A.** Basic formulation OLA73, conventionally drying vs. basic formulation OLA73, accelerated drying **B.** Basic formulation OLA73 vs. formulation including 4% glycerol (OLA100) **C.** Formulation incl. glycerol with water as solvent (OLA100) vs. formulation incl. glycerol with water-ethanol mixture (OLA115) **D.** Basic formulation OLA100 with residual water content of 2% vs. basic formulation OLA100 with residual water content of 6% (designated as OLA108).
- **Figure 3:**: Influence of PEG on the rheological properties of olanzapine ODF (batches MFI149-153; 23°C, 52-53% r.h.)
- **Figure 4:**: Influence of rice starch on the rheological properties of olanzapine ODF (batches MFI145, MFI149; 21-23°C, 49-52% r.h.)
- **Figure 5:**: Influence of glycerol on the rheological properties of olanzapine ODF (batch MFI165; 23°C, 52-53% r.h.)

## Claims

1. An orally disintegrating film (ODF) which comprises
(a) olanzapine or a salt or solvate thereof;
(b) polyvinyl alcohol;
(c) polyethylene glycol
(d) one or more plasticizers;
and whereas at least one of the plasticisers is not PEG1000.

2. Film according to any of the above claims whereas said plasticizer is selected from the group consisting of mono-, di or polysaccharides, polyethers, mono-, di-, tri- or polyvalent alcohols and esters thereof.

3. Film according to claim 9 whereas said plasticizer is sorbitol, glycerol, xanthan, a medium chain triglyceride, polyethylene glycol or carrageenan.

4. Film according claims 1 to 3, wherein the film no more than about 1.0 wt.%, preferably no more than 0.9 wt%, most preferred no more than 0.8 wt % of olanzapine degradation products after the film has been subjected to an accelerated stability test (15 days storage at 60°C).

5. Film according claims 1 to 3, wherein the film comprises no more than about 0.3 wt%, preferably no more than 0.2 wt%, most preferred no more than 0.1 wt% of olanzapine lactam after the film has been subjected to an accelerated stability test (6 days storage at 60°C).

6. Film according to claims 1 to 5 which has a thickness of less between 10 and 500 µm and an area of at least 100mm².

7. Film according to claims 1 to 6 which comprises 1 to 40%, preferably 5 to 30%, more preferably 10 to 25% and most preferred 18 to 22% by weight of olanzapine or a salt or solvate thereof.

8. Film according to claims 1 to 6 which comprises 1 to 40%, preferably 5 to 30%, more preferably 10 to 25% and most preferred 18 to 22% by weight of olanzapine or a salt or solvate thereof.

9. Film according to any of the above claims whereas said polyvinyl alcohol is a polyvinyl alcohol of 20-400 KDa and having a degree of hydrolysis of 85 to 90%, and most preferably a polyvinyl alcohol of about 31 Kda having a degree of hydrolysis of 68.7 to 88.8 %, such as Mowiol 4-88.

10. Film according to any of the above claims whereas said polyethylene glycol polymer (PEG) is a combination of two polyethylene glycol polymers, preferably a combination of at least one solid PEG and at least one liquid PEG (both at room temperature), more preferably a combination of a PEG with a molecular weight between 3000 and 6000 Da and a PEG with a molecular weight between 250 and 1000 Da, and specifically a combination of PEG400 and PEG4000.

11. Film according to any of the above claims which further comprises a disintegrant, preferably starch, more preferably rice starch.

12. Film according to any of the above claims which less than 10% and preferably equal or less than 6% of water by weight.

13. Film according to any of the above claims, wherein the composition comprises no more than about 0.5 wt%, preferably no more than 0.3 wt%, most preferred no more than 0.2 wt % of olanzapine degradation products after the film has been subjected to an accelerated stability test (6 days storage at 60°C).

14. Film according to any of the above claims, wherein the composition comprises no more than about 0.3 wt%, preferably no more than 0.2 wt%, most preferred no more than 0.1 wt% of olanzapine lactam after the film has been subjected to an accelerated stability test (6 days storage at 60°C).

15. Film according to any of the above claims, wherein the film comprises the following components in the given proportions:
(a) 2 to 35 wt% and preferably 5 to 20 wt% olanzapine or a salt or solvate thereof;
(b) 20 to 75 wt% and preferably 30 to 55 wt% polyvinyl alcohol;
(c) 2 to 15 wt% and preferably 5 to 10 wt% polyethylene glycol, preferably a mixture of Macrogol 400 and Macrogol 4000;
(d) 1 to 10 wt% and preferably 2 to 5 wt% plasticizer; preferably rice starch;
(e) 10 to 30 wt% and preferably 15 to 25 wt% disintegrant, preferably rice starch;
and
(f) 1 to 15 wt% of additives.

16. Process for preparing the film according to any of the above claims comprising the following steps:
(i) Preparation of a homogenous solution/dispension of the different components in the presence of a solvent;
(ii) Applying the solution/dispension to a carrier;
(iii) Drying of the applied solution/dispension; and
(iv) Division into films with a defined area.

17. Composition for preparation of a film according to any of the claims 1 to 15, wherein the composition comprises the following components in the given proportions:
(a) 2 to 10 wt% olanzapine or a salt or solvate thereof;
(b) 8 to 18 wt% polyvinyl alcohol, preferably Mowiol 4-88;
(c) 5 to 15 wt% polyethylene glycol, preferably a mixture of Macrogol 400 and Macrogol 4000;
(d) 0.5 to 10 wt% plasticizer, preferably glycerol;
(e) 5 to 15 wt% disintegrant, preferably rice starch;
(f) 0.25 to 5% additives, preferably sweetener and flavouring agent
(g) 40 to 60 wt% water; and
(h) 10 to 20 wt% alcohol, preferably ethanol.

18. Use of the film according to any of the claims 1 to 15 to treat a CNS disorder, preferably psychotic disorders, such as schizophrenia, schizophreniform disorder, mild anxiety and acute mania.
